(19) Europäisches Patentamt
European Patent Office
Office européen des brevets

(11) Numéro de publication : **0 524 055 A1**

(12) **DEMANDE DE BREVET EUROPEEN**

(21) Numéro de dépôt : **92401956.5**

(22) Date de dépôt : **08.07.92**

(51) Int. Cl.5 : **C07D 513/04,** A61K 31/425, // (C07D513/04, 277:00, 235:00)

(30) Priorité : **19.07.91 FR 9109136**
**19.07.91 FR 9109137**
**19.07.91 FR 9109138**
**19.07.91 FR 9109139**

(43) Date de publication de la demande :
**20.01.93 Bulletin 93/03**

(84) Etats contractants désignés :
**AT BE CH DE DK ES FR GB GR IT LI LU MC NL PT SE**

(71) Demandeur : **SYNTHELABO**
**22, Avenue Galilée**
**F-92350 Le Plessis Robinson (FR)**

(72) Inventeur : **George, Pascal**
**19, rue des Quatre Vents**
**F-78730 St Arnoult en Yvelines (FR)**
Inventeur : **de Peretti, Danielle**
**14, rue Ampère**
**F-92160 Antony (FR)**
Inventeur : **Gibert,Jean-François, Résidence Rochebrune Bat.B10**
**10, Impasse Rochebrune**
**F-91220 Bretigny/Orge (FR)**
Inventeur : **Mangane, Michel**
**44, avenue Marcel Cachin**
**F-92320 Chatillon S/Bagneux (FR)**
Inventeur : **Le Galloudec, Odette**
**1, rue au Comte**
**F-91150 Morigny (FR)**

(74) Mandataire : **Thouret-Lemaitre, Elisabeth et al**
**SYNTHELABO, 22, avenue Galilée, B.P. 72**
**F-92350 Le Plessis-Robinson Cédex (FR)**

(54) **Dérivés d'imidazo[2,1-b]benzothiazole-3-acétamide, leur préparation et leur application en thérapeutique.**

(57) Composés répondant à la formule générale (I)

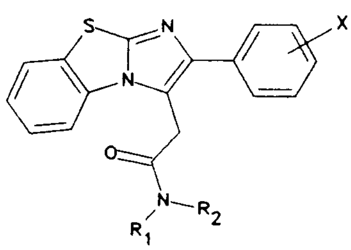

(I)

dans laquelle X représente un atome d'hydrogène ou d'halogène ou un groupe méthyle, éthyle, propyle, méthoxy, éthoxy, méthylthio, méthylsulfonyle, cyano ou aminocarbonyle, $R_1$ représente un atome d'hydrogène ou un groupe alkyle en $C_1$-$C_4$, $R_2$ représente soit un atome d'hydrogène, soit un groupe alkyle en $C_1$-$C_5$ linéaire, ramifié ou cyclique, éventuellement substitué par un ou plusieurs atomes de fluor, par un groupe méthoxy, par un groupe diméthylamino ou par un groupe phényle, soit un groupe prop-2-ényle, soit un groupe prop-2-ynyle, soit un groupe phényle, ou bien $R_1$ et $R_2$ forment ensemble, et avec l'atome d'azote qui les porte, un groupe pyrrolidin-1-yle, pipéridin-1-yle, hexahydroazépin-1-yle, 4-(phénylméthyl)pipéridin-1-yle, 4-méthylpipérazin-1-yle, 4-(phénylméthyl)pipérazin-1-yle, morpholin-1-yle ou thiomorpholin-1-yle. Application en thérapeutique.

EP 0 524 055 A1

La présente invention a pour objet des dérivés d'imidazo[2,1-*b*]benzothiazole-3-acétamide, leur préparation et leur application en thérapeutique.

Les composés de l'invention répondent à la formule générale (I)

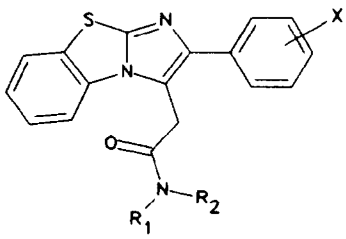

(I)

dans laquelle
X représente un atome d'hydrogène, de fluor, de chlore ou de brome, ou un groupe méthyle, éthyle, propyle, méthoxy, éthoxy, méthylthio, méthylsulfonyle, cyano ou aminocarbonyle,
$R_1$ représente un atome d'hydrogène ou un groupe alkyle en $C_1$-$C_4$, et
$R_2$ représente soit un atome d'hydrogène, soit un groupe alkyle en $C_1$-$C_5$ linéaire, ramifié ou cyclique, éventuellement substitué par un ou plusieurs atomes de fluor, par un groupe méthoxy, par un groupe diméthylamino ou par un groupe phényle,
soit un groupe prop-2-ényle, soit un groupe prop-2-ynyle,
soit un groupe phényle,
ou bien
$R_1$ et $R_2$ forment ensemble, et avec l'atome d'azote qui les porte, un groupe pyrrolidin-1-yle, pipéridin-1-yle, hexahydroazépin-1-yle, 4-(phénylméthyl)pipéridin-1-yle, 4-méthylpipérazin-1-yle, 4-(phénylméthyl)pipérazin-1-yle, morpholin-1-yle ou thiomorpholin-1-yle,
étant exclus les deux composés dans la formule desquels X représente un atome d'hydrogène ou de brome en position 4 et $R_1$ et $R_2$ représentent chacun un atome d'hydrogène, composés décrits comme intermédiaires de synthèses dans *Chem. Pharm. Bull.*, **36**(12), 4760-4768 (1988).

Les composés de l'invention peuvent se présenter à l'état de bases libres ou de sels d'addition à des acides.

Conformément à l'invention, on peut préparer ces composés

Schéma 1

(II)

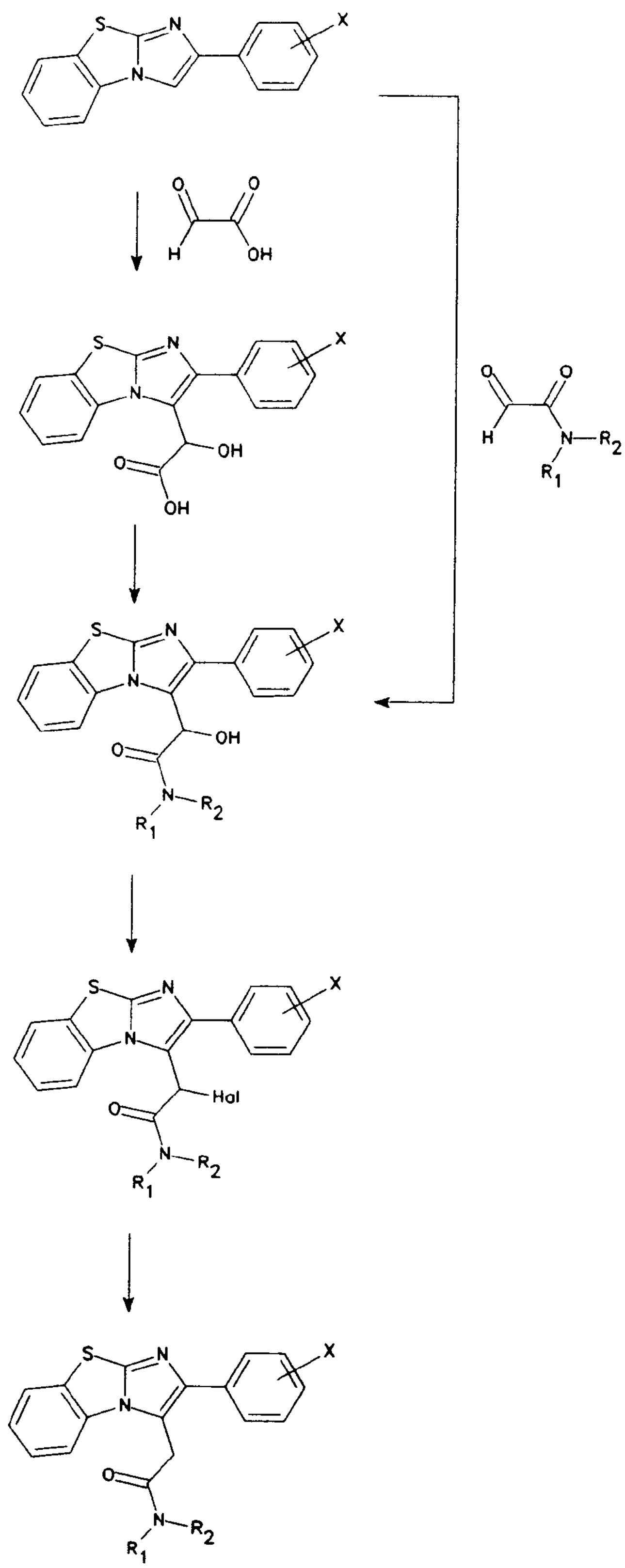

(III)

(IV)

(V)

(I)

selon un procédé illustré par le schéma 1 qui précède.

On fait réagir un dérivé d'imidazo[2,1-*b*]benzothiazole de formule générale (II) (dans laquelle X est tel que défini ci-dessus) avec l'acide glyoxylique dans un solvant protique tel que l'acide acétique, à une température de 20 à 120°C, pour former un dérivé d'acide α-hydroxyacétique de formule générale (III). On fait réagir ce dernier avec l'anhydride acétique en présence d'une base organique telle que la pyridine, à une température de 20 à 60°C, pour former le dérivé d'acide α-acétyloxyacétique correspondant, que l'on traite lui-même au moyen de *N,N'*-carbonyldiimidazole dans un solvant inerte tel qu'un solvant chloré ou éthéré, par exemple le dichlorométhane ou le tétrahydrofurane, à une température de 20 à 50°C, puis on traite *in situ* l'imidazolide intermédiaire ainsi obtenu avec une amine de formule générale $HNR_1R_2$ (dans laquelle $R_1$ et $R_2$ sont tels que définis ci-dessus) à une température de 0 à 20°C.

On obtient un dérivé d'α-hydroxyacétamide de formule générale (IV) qu'on traite ensuite avec un polyhalogénure d'acide sulfurique ou phosphorique, tel que le chlorure de thionyle ou l'oxychlorure de phosphore ou tout autre agent équivalent, dans un solvant inerte tel qu'un solvant chloré ou éthéré, par exemple le dichlorométhane ou le tétrahydrofurane, à une température de 20 à 80°C, pour former un dérivé de α-halogénoacétamide de formule générale (V) (dans laquelle X, $R_1$ et $R_2$ sont tels que définis ci-dessus et Hal représente un atome d'halogène, par exemple le chlore).

Finalement on fait réagir le composé de formule générale (V) soit avec un agent réducteur tel qu'un hydrure alcalin simple ou complexe, par exemple le borohydrure de sodium ou de potassium, dans un solvant protique tel qu'un alcool aliphatique, par exemple le méthanol ou l'éthanol, ou dans un solvant inerte miscible à l'eau, par exemple le dioxane ou le tétrahydrofurane, à une température de -40 à 40°C, soit avec un agent réducteur tel qu'un hyposulfite ou un dithionite alcalin, par exemple l'hyposulfite ou le dithionite de sodium, ou encore avec l'hydroxyméthylsulfoxylate de sodium (Rongalite®), dans un solvant inerte tel qu'un solvant chloré, par exemple le dichlorométhane, éventuellement en présence d'un cosolvant inerte miscible à l'eau, par exemple le *N,N*-diméthylformamide ou la *N*-méthylpyrrolidone, à une température de 20 à 40°C.

Lorsque chacun des symboles $R_1$ et $R_2$ représente un groupe méthyle, une variante du procédé consiste à préparer le dérivé d'α-hydroxyacétamide de formule générale (IV) en traitant le composé de formule générale (II) avec le glyoxamide de formule générale $HC(O)C(O)NR_1R_2$ (dans laquelle $R_1$ et $R_2$ représentent chacun un groupe méthyle et que l'on prépare *in situ* au moyen de 2,2-diéthoxy-*N,N*-diméthylacétamide comme décrit dans la demande de brevet EP-251859), dans un solvant protique tel que l'acide acétique, à une température de 20 à 80°C.

Les composés de formules générales (IV) et(V) sont nouveaux et font partie de l'invention à titre d'intermédiaires dans le procédé de préparation illustré par le schéma 1.

Un autre procédé de préparation est illustré par le schéma 2 ci-après.

Il consiste à faire réagir un dérivé d'acide imidazo[2,1-*b*]-benzothiazole-3-acétique de formule générale (VI) (dans laquelle X est tel que défini ci-dessus) avec un réactif tel que le *N,N'*-carbonyldiimidazole ou le chlorure de thionyle, ou tout autre réactif équivalent, dans un solvant inerte tel qu'un solvant chloré ou éthéré, par exemple le dichlorométhane ou le tétrahydrofurane, à une température de 20 à 50°C, pour obtenir un composé de formule générale (VII) (dans laquelle X est tel que défini ci-dessus et Z représente un atome d'halogène ou un groupe imidazolyle), et finalement on traite ledit composé avec une amine de formule générale $HNR_1R_2$, dans laquelle $R_1$ et $R_2$ sont tels que définis ci-dessus, à une température de 0 à 25°C.

Les dérivés d'imidazo[2,1-*b*]benzothiazole de formule générale (II) peuvent être préparés selon toutes méthodes décrites dans la littérature, par exemple dans *Yakugaku Zasshi,* **60,** 132 (1940) ; *Bull. Soc. Chim. Fr.,* 1277 (1966) ; *J. Indian Chem. Soc.,* **51,** 1031 (1974) ; *J. Med. Chem.,* **29,** 386 (1986).

Schéma 2

(VI)

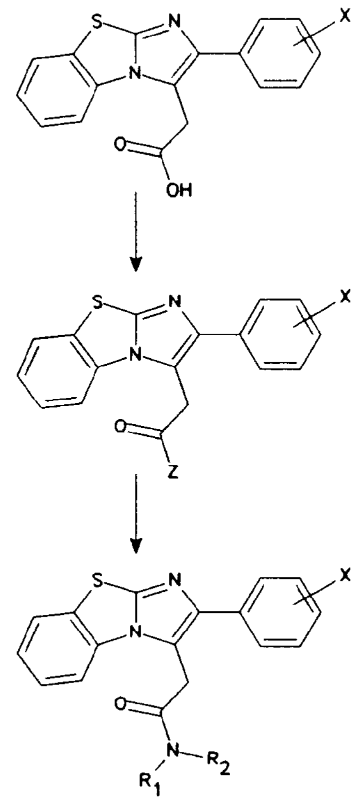

(VII)

(I)

Les dérivés d'acide imidazo[2,1-*b*]benzothiazole-3-acétique de formule générale (VI) peuvent être préparés selon une méthode analogue à celle décrite dans *Chem. Pharm. Bull.*, **36,** 4760 (1988).

Les exemples qui vont suivre illustrent la préparation de quelques composés de l'invention. Les microanalyses élémentaires et les spectres IR et RMN confirment les structures des composés obtenus.
Les numéros indiqués entre parenthèses dans les titres des exemples correspondent à ceux de la première colonne du tableau 1 qui suit.

Exemple 1 (Composé N°1)

*N*-Méthyl-2-phénylimidazo[2,1-*b*]benzothiazole-3-acétamide.

1.1. Acide α-hydroxy-2-phénylimidazo[2,1-*b*]benzothiazole-3-acétique.

On chauffe pendant 6 heures, dans un bain à 120°C, un mélange de 36 g (0,14 mole) de 2-phénylimidazo[2,1-*b*]benzothiazole et 26,5 g (0,28 mole) d'acide glyoxylique dans 1 l d'acide acétique. On évapore le solvant sous pression réduite et on cristallise le résidu dans un mélange d'eau et de dichlorométhane. On collecte le solide par filtration, on le lave à l'eau et on le sèche. On obtient 37,6 g de composé. Point de fusion : 140-143°C.

1.2. α-Hydroxy-*N*-méthyl-2-phénylimidazo[2,1-*b*]benzothiazole-3-acétamide.

On dissout 37,6 g d'acide α-hydroxy-2-phénylimidazo[2,1-*b*]-benzothiazole-3-acétique dans 750 ml d'un mélange 50/50 (v/v) de pyridine et d'anhydride acétique, on agite le mélange pendant une nuit à température ambiante et on évapore le solvant sous pression réduite. On fait cristalliser le résidu dans l'éthanol, on le lave à l'éther diéthylique et on le sèche. On fait réagir 12,2 g (0,033 mole) de l'acide α-acétyloxyacétique ainsi obtenu avec 8,1 g (0,05 mole) de *N,N'*-carbonyldiimidazole dans 160 ml de tétrahydrofurane sec en agitant le mélange

pendant 1,5 heure à température ambiante, puis on le traite par un excès de méthylamine gazeuse sèche. On maintient l'agitation pendant 16 heures puis on évapore le solvant sous pression réduite. On reprend le résidu avec du dichlorométhane et de l'eau, on sépare la phase organique, on la sèche et on évapore le solvant sous pression réduite. On obtient 9,9 g de produit qu'on purifie par chromatographie sur colonne de gel de silice en éluant avec un mélange 98/2 de dichlorométhane/méthanol. On obtient 9,3 g de composé.
Point de fusion : 230-232°C.

1.3. α-Chloro-*N*-méthyl-2-phénylimidazo[2,1-*b*]benzothiazole-3-acétamide, chlorhydrate.

On traite 9,2 g (0,027 mole) de α-hydroxy-*N*-méthyl-2-phénylimidazo[2,1-*b*]benzothiazole-3-acétamide dans 200 ml de dichlorométhane sec avec 50 ml de chlorure de thionyle pendant 16 heures à température ambiante. On évapore le solvant sous pression réduite, on fait cristalliser le résidu dans l'éther diéthylique et on le sèche. On obtient 10,2 g de chlorhydrate qu'on utilise tel quel dans l'étape suivante.

1.4. *N*-Méthyl-2-phénylimidazo[2,1-*b*]benzothiazole-3-acétamide.

On traite 9,8 g (0,025 mole) de chlorhydrate de α-chloro-*N*-méthyl-2-phénylimidazo[2,1-*b*]benzothiazole-3-acétamide dans 200 ml de dichlorométhane avec 11,6 g (0,075 mole) de Rongalite® pendant 24 heures à température ambiante. On filtre la suspension, on lave le filtrat avec une solution bicarbonatée, on le sèche sur sulfate de sodium, on le filtre et on l'évapore sous pression réduite. On purifie le résidu par chromatographie sur colonne de gel de silice en éluant avec un mélange 99/1 de dichlorométhane/méthanol et on le recristallise dans l'éthanol.
On obtient finalement 4,1 g de composé.
Point de fusion : 260-263°C.

Exemple 2 (Composé N°20)

2-(4-Méthoxyphényl)-*N,N*-diméthylimidazo[2,1-*b*]benzothiazole-3-acétamide.

2.1. Acide α-hydroxy-2-(4-méthoxyphényl)imidazo[2,1-*b*]benzothiazole-3-acétique.

On chauffe pendant 5,5 heures, dans un bain à 80°C, un mélange de 50 g (0,178 mole) de 2-(4-méthoxyphényl)imidazo-[2,1-*b*]benzothiazole, 32,8 g (0,356 mole) d'acide glyoxylique et 1200 ml d'acide acétique. On évapore le solvant sous pression réduite et on fait cristalliser le résidu dans un mélange d'eau et de dichlorométhane. On sépare l'insoluble par filtration, on le lave à l'eau, puis à l'éthanol, puis à l'éther diéthylique. On obtient 34,65 g de composé.
Point de fusion : 160-163°C.

2.2. α-Hydroxy-2-(4-méthoxyphényl)-*N,N*-diméthylimidazo-[2,1-*b*]benzothiazole-3-acétamide.

Dans 480 ml d'un mélange 50/50 (v/v) de pyridine et d'anhydride acétique on dissout 34,65 g d'acide α-hydroxy-2-(4-méthoxyphényl)imidazo[2,1-*b*]benzothiazole-3-acétique et on agite le mélange pendant une nuit à température ambiante. On évapore le solvant sous pression réduite, on fait cristalliser le résidu dans l'éthanol, on lave les cristaux à l'éther diéthylique et on les sèche.
On agite un mélange de 7,5 g (0,019 mole) de l'acide α-acétyloxyacétique ainsi obtenu, 4,32 g (0,027 mole) de *N,N'*-carbonyldiimidazole et 90 ml de tétrahydrofurane sec pendant 1,5 heure à température ambiante. On refroidit le mélange dans un bain de glace et on le traite avec un excès de diméthylamine gazeuse sèche. On maintient l'agitation pendant 16 heures à température ambiante, et on évapore le solvant sous pression réduite. On reprend le résidu avec de l'eau, on sépare l'insoluble par filtration; on le lave à l'eau, à l'éthanol, puis à l'éther diéthylique. Après recristallisation dans l'éthanol on obtient 3,08 g de composé.
Point de fusion : 225-227°C.

2.3. α-Chloro-2-(4-méthoxyphényl)-*N,N*-diméthylimidazo-[2,1-*b*]benzothiazole-3-acétamide, chlorhydrate.

On traite 2,95 g de α-hydroxy-2-(4-méthoxyphényl)-*N,N*-diméthylimidazo[2,1-*b*]benzothiazole-3-acétamide dans 60 ml de dichlorométhane sec avec 15 ml de chlorure de thionyle pendant 16 heures à température ambiante. On évapore le solvant sous pression réduite, on fait cristalliser le résidu dans l'éther diéthylique, et on obtient 3,13 g de chlorhydrate qu'on utilise tel quel dans l'étape suivante.

2.4. 2-(4-Méthoxyphényl)-*N,N*-diméthylimidazo[2,1-*b*]benzothiazole-3-acétamide.

On traite 3,0 g (0,007 mole) de chlorhydrate de α-chloro-2-(4-méthoxyphényl)-*N,N*-diméthylimidazo[2,1-*b*]benzothiazole-3-acétamide avec 3,34 g (0,022 mole) de Rongalite® dans 50 ml de dichlorométhane et 40 ml de *N,N*-diméthylformamide pendant 24 heures à température ambiante. On filtre la suspension, on évapore le filtrat sous pression réduite, on reprend le résidu avec de l'eau et du dichlorométhane, on sépare la phase organique, on la lave avec de l'eau bicarbonatée puis avec de l'eau, on la sèche et on évapore le solvant sous pression réduite. On recristallise le résidu dans l'éthanol et on isole finalement 1,62 g de composé.
Point de fusion : 210-213°C.

Exemple 3 (Composé N°15)

*N*-Méthyl-2-(3-méthylphényl)imidazo[2,1-*b*]benzothiazole-3-acétamide.

3.1. Acide α-hydroxy-2-(3-méthylphényl)imidazo[2,1-*b*]benzothiazole-3-acétique.

On agite pendant 2 jours à température ambiante un mélange de 19 g (0,072 mole) de 2-(3-méthylphényl)imidazo[2,1-*b*]benzothiazole et 13,2 g (0,143 mole) d'acide glyoxylique dans 450 ml d'acide acétique. On évapore le solvant sous pression réduite et on fait cristalliser le résidu dans un mélange d'eau et de dichlorométhane. On filtre la suspension obtenue, on lave le solide à l'eau, à l'éthanol puis à l'éther diéthylique. Après séchage sous vide on obtient 23,7 g de composé.
Point de fusion : 173-176°C.

3.2. α-Hydroxy-*N*-méthyl-2-(3-méthylphényl)imidazo[2,1-*b*]benzothiazole-3-acétamide.

On dissout 23 g (0,068 mole) d'acide α-hydroxy-2-(3-méthylphényl)imidazo[2,1-*b*]benzothiazole-3-acétique dans 440 ml d'un mélange 50/50 (v/v) de pyridine et d'acide acétique, on agite le mélange à température ambiante pendant 16 heures, puis on évapore les solvants sous pression réduite.
On agite un mélange de 9,33 g (0,024 mole) de l'acide α-acétyloxyacétique huileux ainsi obtenu et 5,37 g (0,033 mole) de *N,N'*-carbonyldiimidazole dans 175 ml de tétrahydrofurane sec pendant 1,5 heure à température ambiante. On refroidit le mélange avec un bain d'eau et de glace, et on le traite avec un excès de méthylamine gazeuse sèche. On maintient l'agitation à température ambiante pendant 16 heures, puis on évapore le solvant sous pression réduite. On reprend le résidu avec de l'eau et du dichlorométhane, on sépare la phase organique, on la sèche et on évapore le solvant sous pression réduite.
On obtient 7,3 g de produit qu'on purifie par chromatographie sur colonne de gel de silice en éluant avec un mélange 99/1 de dichlorométhane/méthanol. On obtient 5,3 g de composé.
Point de fusion : 179-182°C.

3.3. α-Chloro-*N*-méthyl-2-(3-méthylphényl)imidazo[2,1-*b*]benzothiazole-3-acétamide, chlorhydrate.

On traite 4,6 g (0,013 mole) de α-hydroxy-*N*-méthyl-2-(3-méthylphényl)imidazo[2,1-*b*]benzothiazole-3-acétamide avec 24 ml de chlorure de thionyle dans 100 ml de dichlorométhane pendant 16 heures à température ambiante. On évapore le solvant sous pression réduite et on fait cristalliser le résidu dans l'éther diéthylique. On obtient 5,17 g de chlorhydrate qu'on utilise tel quel dans l'étape suivante.

3.4. *N*-Méthyl-2-(3-méthylphényl)imidazo[2,1-*b*]benzothiazole-3-acétamide.

On traite 5,17 g (0,013 mole) du chlorhydrate de α-chloro-*N*-méthyl-2-(3-méthylphényl)imidazo[2,1-*b*]benzothiazole-3-acétamide avec 5,9 g (0,038 mole) de Rongalite® dans 90 ml de dichlorométhane et 70 ml de *N,N*-diméthylformamide à température ambiante pendant 24 heures.
On filtre la suspension obtenue, on évapore le filtrat sous pression réduite, on reprend le résidu avec de l'eau et du dichlorométhane, on sépare la phase organique, on la lave à l'eau bicarbonatée puis à l'eau, on la sèche et on évapore le solvant sous pression réduite. Après purification du résidu par recristallisation dans l'éthanol on isole finalement 2,37 g de composé.
Point de fusion : 201-203°C.

Exemple 4 (Composé N°6)

2-(2-Chlorophényl)-*N,N*-diméthylimidazo[2,1-*b*]benzothiazole-3-acétamide.

4.1. Acide 2-(2-chlorophényl)-α-hydroxyimidazo[2,1-*b*]benzothiazole-3-acétique.

On chauffe pendant 3,5 heures, dans un bain à 80°C, un mélange de 27,7 g (0,097 mole) de 2-(2-chlorophényl)imidazo-[2,1-*b*]benzothiazole et 14,4 g (0,194 mole) d'acide glyoxylique dans 500 ml d'acide acétique. On évapore la solution sous pression réduite, on reprend le résidu avec de l'eau, on collecte le solide par filtration, on le lave à l'eau, on le dissout dans de l'éthanol et on concentre la solution sous pression réduite. On lave le résidu avec de l'éther diéthylique et on le sèche sous vide. On obtient 32,6 g de composé. Point de fusion : 180-184°C.

4.2. 2-(2-Chlorophényl)-α-hydroxy-*N,N*-diméthylimidazo-[2,1-*b*]benzothiazole-3-acétamide.

On agite un mélange de 32,6 g (0,091 mole) d'acide 2-(2-chlorophényl)-α-hydroxyimidazo[2,1-*b*]benzothiazole-3-acétique, 400 ml de pyridine et 400 ml d'acide acétique pendant 16 heures à température ambiante. On le concentre sous pression réduite, on reprend le résidu avec de l'éther diéthylique, on collecte le solide par filtration et on le sèche sous vide. On fait réagir 11 g (0,027 mole) de l'acide α-acétyloxyacétique ainsi obtenu avec 4,9 g (0,030 mole) de *N,N'*-carbonyldiimidazole dans 140 ml de tétrahydrofurane sec, on agite le mélange pendant 1 heure à température ambiante puis on le refroidit avec un bain de glace et on le traite avec un excès de diméthylamine gazeuse sèche.
On maintient l'agitation pendant 16 heures à température ambiante, puis on évapore le solvant sous pression réduite. On reprend le résidu avec du dichlorométhane et de l'eau, on sépare la phase organique, on la lave à l'eau jusqu'à pH neutre, on la sèche sur sulfate de sodium, on évapore le solvant sous pression réduite, on lave le résidu avec de l'éther diéthylique et on le sèche. On obtient 6,7 g de composé. Point de fusion : 203°C.

4.3. α-Chloro-2-(2-chlorophényl)-*N,N*-diméthylimidazo[2,1-*b*]-benzothiazole-3-acétamide, chlorhydrate.

On traite 6,4 g (0,016 mole) de 2-(2-chlorophényl)-α-hydroxy-*N,N*-diméthylimidazo[2,1-*b*]benzothiazole-3-acétamide dans 340 ml de dichlorométhane sec avec 34 ml de chlorure de thionyle pendant 16 heures à température ambiante, puis on évapore le solvant et l'excès de chlorure de thionyle sous pression réduite. On lave le résidu solide avec de l'éther diéthylique sec et on le sèche sous vide. On obtient 7,0 g de composé.

4.4. 2-(2-Chlorophényl)-*N,N*-diméthylimidazo[2,1-*b*]benzothiazole-3-acétamide.

On traite 7,0 g (0,016 mole) de chlorhydrate de α-chloro-2-(2-chlorophényl)-*N,N*-diméthylimidazo[2,1-*b*]benzothiazole-3-acétamide avec 7,3 g (0,047 mole) de Rongalite® dans 320 ml d'un mélange 50/50 (v/v) de dichlorométhane et de *N,N*-diméthylformamide pendant 40 heures à température ambiante. On filtre la suspension, on concentre le filtrat sous pression réduite, on reprend le résidu avec de l'eau, on collecte le solide par filtration, on le lave à l'eau bicarbonatée puis à l'eau jusqu'à pH neutre, on le dissout dans de l'éthanol, on évapore l'éthanol sous pression réduite, on fait cristalliser le résidu dans de l'éther diéthylique, on le sèche sous vide, on le purifie par chromatographie sur colonne de gel de silice en éluant avec un mélange 98/2 de dichlorométhane/méthanol et on le recristallise dans l'acétate d'éthyle. On obtient 1,5 g de composé.
Point de fusion : 159-162°C.

Exemple 5 (Composé N°4)

2-(4-Fluorophényl)-*N,N*-diméthylimidazo[2,1-*b*]benzothiazole-3-acétamide.

5.1. 2-(4-Fluorophényl)-α-hydroxy-*N,N*-diméthylimidazo-[2,1-*b*]benzothiazole-3-acétamide.

Dans un ballon de 1000 ml on mélange 39,2 g (0,22 mole) de 2,2-diéthoxy-*N,N*-diméthylacétamide et 55 ml d'acide chlorhydrique à 37% dilués dans 370 ml d'acide acétique, et on chauffe le mélange en l'agitant dans un bain à 60°C pendant 1,5 heure.
On ajoute ensuite 18,3 g (0,22 mole) d'acétate de sodium puis, par petites portions, 20 g (0,074 mole) de 2-(4-fluorophényl)imidazo[2,1-*b*]benzothiazole, et on maintient le chauffage et l'agitation pendant 2 heures.
On évapore le mélange sous pression réduite, on reprend le résidu avec de l'eau et du dichlorométhane, on

élimine un insoluble par filtration, on sépare la phase organique, on la lave à l'eau bicarbonatée et on la sèche sur sulfate de sodium. On évapore le solvant sous pression réduite, et on purifie le résidu par chromatographie sur colonne de gel de silice en éluant avec un mélange 97/3 de dichlorométhane/méthanol, puis par cristallisation dans l'éther diéthylique. On obtient 7,7 g de composé.
Point de fusion : 212-215°C.

5.2. α-Chloro-2-(4-fluorophényl)-*N,N*-diméthylimidazo[2,1-*b*]-benzothiazole-3-acétamide, chlorhydrate.

On traite 7,6 g (0,02 mole) de 2-(4-fluorophényl)-α-hydroxy-*N,N*-diméthylimidazo[2,1-*b*]benzothiazole-3-acétamide avec 40 ml de chlorure de thionyle dans 400 ml de dichlorométhane à température ambiante pendant 16 heures, on évapore le solvant et l'excès de chlorure de thionyle sous pression réduite. On reprend le résidu dans de l'éther diéthylique sec, on le filtre et on le sèche. On obtient 7,9 g de composé.

5.3. 2-(4-Fluorophényl)-*N,N*-diméthylimidazo[2,1-*b*]benzothiazole-3-acétamide.

On traite 7,9 g (0,019 mole) de chlorhydrate de α-chloro-2-(4-fluorophényl)-*N,N*-diméthylimidazo[2,1-*b*]benzothiazole-3-acétamide dans 360 ml d'un mélange 50/50 (v/v) de dichlorométhane et de *N,N*-diméthylformamide avec 8,6 g (0,056 mole) de Rongalite® pendant 18 heures à température ambiante. On filtre la suspension, on évapore le filtrat sous pression réduite et on traite le résidu d'évaporation avec une solution aqueuse de bicarbonate de sodium. On collecte le solide par filtration, on le lave à l'eau jusqu'à pH neutre, puis à l'éthanol et à l'éther diéthylique, et on le sèche sous vide.

Pour finir on le purifie par chromatographie sur colonne de gel de silice en éluant avec un mélange 98/2 de dichlorométhane/méthanol et on le recristallise dans l'éthanol. On obtient 3,0 g de composé.
Point de fusion : 230-231°C.

Exemple 6 (Composé N°9)

2-(4-Chlorophényl)-*N*-méthylimidazo[2,1-*b*]benzothiazole-3-acétamide.

On traite 1 g (0,003 mole) d'acide 2-(4-chlorophényl)imidazo[2,1-*b*]benzothiazole-3-acétique avec 0,54 g (0,0033 mole) de *N,N'*-carbonyldiimidazole dans 15 ml de tétrahydrofurane sec pendant 1 heure à température ambiante.
On refroidit le mélange par un bain de glace, et on le traite avec un excès de méthylamine gazeuse sèche. On agite le mélange pendant 15 heures à température ambiante, on le concentre sous vide, on collecte le solide par filtration, on le lave à l'eau jusqu'à pH neutre, puis à l'éthanol et à l'éther diéthylique, on le sèche et on le purifie par recristallisation dans l'éthanol. On obtient 0,57 g de composé.
Point de fusion : 277-278°C (décomp.).

Exemple 7 (Composé N°21)

2-Phénylimidazo[2,1-*b*]benzothiazole-3-acétamide.

7.1. Acide α-hydroxy-2-phénylimidazo[2,1-*b*]benzothiazole-3-acétique.

On chauffe pendant 6 heures, dans un bain à 120°C, un mélange de 36 g (0,144 mole) de 2-phénylimidazo[2,1-*b*]benzothiazole, 1000 ml d'acide acétique et 26,5 g (0,288 mole) d'acide glyoxylique.
On évapore le solvant, on reprend le résidu avec de l'éther diéthylique, on collecte les cristaux par filtration et on les sèche. On obtient 37,63 g de composé.

7.2. α-Hydroxy-2-phénylimidazo[2,1-*b*]benzothiazole-3-acétamide.

On agite pendant 16 heures à température ambiante un mélange de 37,6 g (0,116 mole) d'acide α-hydroxy-2-phénylimidazo-[2,1-*b*]benzothiazole-3-acétique, 380 ml de pyridine et 380 ml d'anhydride acétique.
On évapore les solvants sous pression réduite, on reprend le résidu deux fois avec du toluène, qu'on évapore, on reprend le résidu avec de l'éthanol, on collecte le solide par filtration, on le lave à l'éther diéthylique et on le sèche. On obtient 31,17 g de dérivé α-acétyloxyacétique intermédiaire dont on dissout 12,2 g (0,033 mole) dans 160 ml de tétrahydrofurane, on ajoute, par fractions, 8,1 g (0,050 mole) de *N,N'*-carbonyldiimidazole et on maintient l'agitation pendant 1,5 heure, puis on traite le mélange avec un courant d'ammoniac sec pendant

1,5 heure. On maintient l'agitation pendant 16 heures, on ajoute de l'eau et on évapore le tétrahydrofurane sous pression réduite, on reprend le résidu avec du dichlorométhane, on collecte le solide par filtration, on le sèche, on le traite avec un mélange de 100 ml de méthanol, 100 ml d'eau et 20 g de carbonate de potassium, on collecte le solide par filtration, on le lave à l'eau puis à l'éther diéthylique. Après séchage on obtient 6,59 g de composé.
Point de fusion : 239-241°C.

7.3. α-Chloro-2-phénylimidazo[2,1-*b*]benzothiazole-3-acétamide, chlorhydrate.

A une solution de 6,45 g (0,020 mole) d'α-hydroxy-2-phénylimidazo[2,1-*b*]benzothiazole-3-acétamide dans 150 ml de dichlorométhane on ajoute, goutte à goutte, 36 ml (0,5 mole) de chlorure de thionyle et on agite le mélange pendant 16 heures. On évapore le dichlorométhane sous pression réduite, on chasse l'excès de chlorure de thionyle avec du toluène, et on lave le solide obtenu avec de l'éther diéthylique. On obtient 6,84 g de chlorhydrate.
Rendement : 90%.

7.4. 2-Phénylimidazo[2,1-*b*]benzothiazole-3-acétamide.

On agite à température ambiante, pendant 16 heures, un mélange de 6,5 g (0,0159 mole) de chlorhydrate d'α-chloro-2-phénylimidazo[2,1-*b*]benzothiazole-3-acétamide, 150 ml de dichlorométhane et 7,94 g (0,048 mole) de Rongalite®.

On ajoute une solution aqueuse de bicarbonate de sodium, on collecte le solide par filtration, on le lave à l'eau, puis à l'éthanol puis à l'éther diéthylique, et on le recristallise dans l'éthanol puis deux fois dans le méthanol.
On obtient finalement 0,81 g de composé.
Point de fusion : 246-248°C.

Exemple 8 (Composé N°22)

2-(4-Bromophényl)imidazo[2,1-*b*]benzothiazole-3-acétamide.

8.1. Acide 2-(4-bromophényl)-α-hydroxyimidazo[2,1-*b*]benzothiazole-3-acétique.

On chauffe pendant 3,5 heures, dans un bain à 80°C, un mélange de 33 g (0,1 mole) de 2-(4-bromophényl)imidazo-[2,1-*b*]benzothiazole, 650 ml d'acide acétique et 18,45 g (0,2 mole) d'acide glyoxylique.
On évapore le solvant, on reprend le résidu avec du dichlorométhane et de l'eau, on collecte le solide par filtration, on le lave à l'eau, puis à l'éthanol, puis à l'éther diéthylique. Après séchage on obtient 36,41 g de composé.
Rendement : 90%.

8.2. 2-(4-Bromophényl)-α-hydroxyimidazo[2,1-*b*]benzothiazole-3-acétamide.

On agite pendant 16 heures à température ambiante un mélange de 47 g (0,116 mole) d'acide 2-(4-bromophényl)-α-hydroxyimidazo[2,1-*b*]benzothiazole-3-acétique, 380 ml de pyridine et 380 ml d'anhydride acétique.
On évapore les solvants sous pression réduite, on reprend le résidu deux fois avec du toluène, qu'on évapore, on reprend le résidu avec de l'éthanol, on collecte le solide par filtration, on le lave à l'éther diéthylique et on le sèche. On obtient 46,69 g de dérivé α-acétyloxyacétique intermédiaire dont on dissout 15,3 g (0,034 mole) dans 160 ml de tétrahydrofurane, on ajoute, par fractions, 8,36 g (0,051 mole) de *N,N'*-carbonyldiimidazole et on maintient l'agitation pendant 1,5 heure, puis on traite le mélange avec un courant d'ammoniac sec pendant 1,5 heure. On maintient l'agitation pendant 16 heures, on ajoute de l'eau et on évapore le tétrahydrofurane sous pression réduite, on reprend le résidu avec de l'eau, on collecte le solide par filtration, on le lave à l'eau, puis à l'éthanol puis à l'éther diéthylique, on le sèche, on le traite avec un mélange de 120 ml de méthanol, 120 ml d'eau et 25 g de carbonate de potassium, on collecte le solide par filtration, on le lave à l'eau puis à l'éther diéthylique. Après séchage on obtient 9,41 g de composé.
Point de fusion : 254-257°C.

8.3. 2-(4-Bromophényl)-α-chloroimidazo[2,1-*b*]benzothiazole-3-acétamide, chlorhydrate.

A une solution de 9 g (0,022 mole) de 2-(4-bromophényl)-α-hydroxyimidazo[2,1-*b*]benzothiazole-3-acétamide dans 180 ml de dichlorométhane on ajoute, goutte à goutte, 55 ml (0,56 mole) de chlorure de thionyle et on agite le mélange pendant 16 heures. On évapore le dichlorométhane sous pression réduite, on chasse l'excès de chlorure de thionyle avec du toluène, et on lave le solide obtenu avec de l'éther diéthylique. On obtient 9,39 g de chlorhydrate.

8.4. 2-(4-Bromophényl)imidazo[2,1-*b*]benzothiazole-3-acétamide.

On agite à température ambiante, pendant 16 heures, un mélange de 9 g (0,0197 mole) de chlorhydrate de 2-(4-bromophényl)-α-chloroimidazo[2,1-*b*]benzothiazole-3-acétamide, 160 ml de dichlorométhane, 160 ml de *N,N*-diméthylformamide et 9,1 g (0,059 mole) de Rongalite®.
On filtre le mélange, on lave le solide avec une solution aqueuse saturée de bicarbonate de sodium, puis avec de l'eau, puis avec de l'éthanol, puis avec de l'éther diéthylique. On obtient 4,15 g de produit. Par ailleurs on évapore le filtrat, on reprend le résidu avec de l'eau, on collecte le solide par filtration, on le lave successivement avec de l'eau, une solution saturée de bicarbonate de sodium, encore de l'eau, de l'éthanol, puis de l'éther diéthylique. Après séchage on obtient encore 1,92 g de produit qu'on ajoute au précédent, soit un total de 6,07 g. Après recristallisation dans le méthanol on isole finalement 2,02 g de composé.
Point de fusion : 287-290°C.

Exemple 9 (Composé N°29)

*N*-(Prop-2-ényl)-2-phénylimidazo[2,1-*b*]benzothiazole-3-acétamide.

Sous atmosphère d'azote on ajoute 1,77 g (0,011 mole) de *N,N'*-carbonyldiimidazole à une suspension de 2,5 g (0,008 mole) d'acide 2-phénylimidazo[2,1-*b*]benzothizole-3-acétique dans 40 ml de tétrahydrofurane sec. On laisse agiter 2,5 heures à température ambiante puis on ajoute 0,69 ml (0,0089 mole) de prop-2-énylamine et on continue l'agitation pendant 4 heures. On évapore le solvant sous pression réduite, on reprend le résidu avec de l'eau et du dichlorométhane. On lave la phase organique à l'eau et on la sèche sur sulfate de sodium. Après évaporation du solvant sous pression réduite on obtient 2,33 g de résidu que l'on purifie par chromatographie sur colonne de gel de silice en éluant par un mélange 99/1 de dichlorométhane/méthanol, puis par recristallisation dans l'éthanol. On obtient 1,65g de composé.
Point de fusion : 210-212°C.

Exemple 10 (Composé N°42)

*N*-Éthyl-2-(4-méthoxyphényl)imidazo[2,1-*b*]benzothiazole-3-acétamide.

Sous atmosphère d'azote on ajoute 1,44 g (0,0088 mole) de *N,N'*-carbonyldiimidazole à une suspension de 2 g (0,0059 mole) d'acide 2-(4-méthoxyphényl)imidazo[2,1-*b*]benzothiazole-3-acétique dans 45 ml de tétrahydrofurane sec. On laisse agiter le mélange réactionnel pendant 1,5 heure à température ambiante, puis on refroidit au bain de glace, on introduit 5 ml d'éthylamine liquide anhydre et on laisse agiter pendant 18 heures à température ambiante.
On évapore le solvant sous pression réduite et on reprend le résidu à l'eau. On sépare l'insoluble par filtration, on le lave à l'eau à l'éthanol puis à l'éther diéthylique. On purifie le composé ainsi obtenu par recristallisation dans l'éthanol puis par chromatographie sur colonne de gel de silice en éluant avec un mélange 99/1 de dichlorométhane/méthanol. Après cristallisation dans l'éther diéthylique, on obtient 1,16 g de composé.
Point de fusion : 224-225°C.

Exemple 11 (Composé N°55)

1-[(2-Phénylimidazo[2,1*b*]benzothiazol-3-yl)acétyl]-4-méthylpipérazine.

A une suspension de 3 g (0,0097 mole) d'acide 2-phénylimidazo[2,1-*b*]benzothiazole-3-acétique dans 100 ml de dichlorométhane sec, on ajoute 2,35 g (0,0146 mole) de *N,N'*-carbonyldiimidazole et on laisse agiter 10 minutes à température ambiante. A la solution obtenue, on ajoute 0,97 g (0,0097 mole) de *N*-méthylpipérazine en solution dans 10 ml de dichlorométhane. On agite le mélange réactionnel pendant 6 heures à température

ambiante puis on le verse dans 100 ml d'eau. On décante la phase organique, on la lave à l'eau et on la sèche sur sulfate de sodium. Après évaporation du solvant sous pression réduite, on obtient 2,73 g d'une huile qui cristallise dans l'acétate d'éthyle. On la purifie en préparant le chlorhydrate à l'aide d'une solution d'éther chlorhydrique, et on le recristallise dans le propanol. On décompose le chlorhydrate avec une solution d'ammoniaque diluée, et on extrait la base ainsi obtenue avec du dichlorométhane. On lave la phase organique à l'eau, on la sèche, on évapore le solvant et on recristallise le résidu dans l'acétate d'éthyle. On obtient 2,2 g de composé.
Point de fusion : 195-196°C.

Exemple 12 (Composé N°59)

1-[[2-(4-Chlorophényl)imidazo[2,1*b*]benzothiazol-3-yl]acétyl]pyrrolidine.

A une suspension de 2 g (0,0059 mole) d'acide 2-(4-chlorophényl)imidazo[2,1-*b*]benzothiazole-3-acétique dans 50 ml de dichlorométhane sec, on ajoute 1,4 g (0,0087 mole) de *N,N'*-carbonyldiimidàzole et on laisse agiter le mélange réactionnel pendant 10 minutes à température ambiante. A la solution ainsi obtenue, on ajoute 0,42 g (0,0058 mole) de pyrrolidine en solution dans 5 ml de dichlorométhane. On agite le mélange réactionnel pendant 6 heures à température ambiante, puis on le verse dans 50 ml d'eau. On décante la phase organique, on la lave à l'eau et on la sèche sur sulfate de sodium. Après évaporation du solvant sous pression réduite, on fait cristalliser le résidu dans le propan-2-ol. On obtient 1,9 g de composé.
Point de fusion : 248-249°C.

Exemple 13 (Composé N°72)

2-(3-méthylphényl)imidazo[2,1-*b*]benzothiazole-3-acétamide.

13.1. Acide α-hydroxy-2-(3-méthylphényl)imidazo[2,1-*b*]benzothiazole-3-acétique.

On agite pendant 2 jours à température ambiante un mélange de 19 g (0,072 mole) de 2-(3-méthylphényl)imidazo[2,1-*b*]benzothiazole et 13,2 g (0,143 mole) d'acide glyoxylique dans 450 ml d'acide acétique. On évapore le solvant sous pression réduite et on fait cristalliser le résidu dans un mélange d'eau et de dichlorométhane. On filtre la suspension obtenue, on lave le solide à l'eau, à l'éthanol puis à l'éther diéthylique. Après séchage sous vide on obtient 23,7 g de composé.
Point de fusion : 173-176°C.

13.2. α-hydroxy-2-(3-méthylphényl)imidazo[2,1-*b*]benzothiazole-3-acétamide.

On dissout 23 g (0,068 mole) d'acide α-hydroxy-2-(3-méthylphényl)imidazo[2,1-*b*]benzothiazole-3-acétique dans 440 ml d'un mélange 50/50 (v/v) de pyridine et d'acide acétique, on agite le mélange à température ambiante pendant 16 heures, puis on évapore les solvants sous pression réduite.
On agite un mélange de 9,33 g (0,024 mole) de l'acide α-acétyloxyacétique huileux ainsi obtenu et 5,37 g (0,033 mole) de *N,N'*-carbonyldiimidazole dans 175 ml de tétrahydrofurane sec pendant 1,5 heure à température ambiante. On refroidit le mélange avec un bain d'eau et de glace, et on le traite avec un excès d'ammoniac gazeux sec. On maintient l'agitation à température ambiante pendant 16 heures, puis on évapore le solvant sous pression réduite. On reprend le résidu avec de l'eau et du dichlorométhane, on filtre la suspension obtenue, on lave le solide à l'eau, à l'éthanol puis à l'éther diéthylique. On obtient 6,1 g d'α-acétyloxy-2-(3-méthylphényl)imidazo[2,1-*b*]benzothiazole-3-acétamide qu'on traite avec 12 g de carbonate de potassium dans 300 ml d'un mélange 50/50 (v/v) d'eau et de méthanol, en agitant le mélange pendant 100 heures à température ambiante.
On sépare le solide par filtration, et on le lave plusieurs fois à l'eau, à l'éthanol puis à l'éther diéthylique. On obtient 5,4 g de composé.
Point de fusion : 253-255°C.

13.3. α-chloro-2-(3-méthylphényl)imidazo[2,1-*b*]benzothiazole-3-acétamide, chlorhydrate.

On traite 5,2 g (0,015 mole) de α-hydroxy-2-(3-méthylphényl)-imidazo[2,1-*b*]benzothiazole-3-acétamide avec 28 ml de chlorure de thionyle dans 110 ml de dichlorométhane pendant 16 heures à température ambiante. On évapore le solvant sous pression réduite et on fait cristalliser le résidu dans l'éther diéthylique. On obtient 5,7 g de chlorhydrate qu'on utilise tel quel dans l'étape suivante.

13.4. 2-(3-méthylphényl)imidazo[2,1-*b*]benzothiazole-3-acétamide.

On traite 5,7 g (0,014 mole) du chlorhydrate de α-chloro-2-(3-méthylphényl)imidazo[2,1-*b*]benzothiazole-3-acétamide avec 6,7 g (0,044 mole) de Rongalite® dans 100 ml de dichlorométhane et 80 ml de *N,N*-diméthylformamide à température ambiante pendant 24 heures. On filtre la suspension obtenue, on évapore le filtrat, on reprend le résidu avec de l'eau, on filtre le solide obtenu, on le lave avec une solution saturée de bicarbonate de sodium, avec de l'eau, avec de l'éthanol, puis avec de l'éther diéthylique. On obtient 2,1 g de composé qu'on recristallise dans l'éthanol puis dans le méthanol. On isole finalement 1,03 g de composé pur. Point de fusion : 243-246°C.

Exemple 14 (Composé N°70)

2-(4-chlorophényl)imidazo[2,1-*b*]benzothiazole-3-acétamide.

On traite 1 g (0,003 mole) d'acide 2-(4-chlorophényl)imidazo[2,1-*b*]benzothiazole-3-acétique avec 0,54 g (0,0033 mole) de *N,N'*-carbonyldiimidazole dans 15 ml de tétrahydrofurane sec pendant 1 heure à température ambiante.
On refroidit le mélange par un bain de glace, et on le traite avec un excès d'ammoniac gazeux sec. On agite le mélange pendant 15 heures à température ambiante, on le concentre sous vide, on collecte le solide par filtration, on le lave à l'eau jusqu'à pH neutre, puis à l'éthanol et à l'éther diéthylique, on le sèche et on le purifie par recristallisation dans l'éthanol. On obtient 0,63 g de composé.
Point de fusion : 288-290°C (décomp.).

Le tableau 1 qui suit illustre les structures chimiques et les propriétés physiques de quelques composés de formule générale (I). Tous les composés sont à l'état de base, à l'exception du N°32, qui est un chlorhydrate, et du N°90, qui est un hydrate.
Dans la colonne "F (°C), "(d)" désigne un point de fusion avec décomposition.

Tableau 1

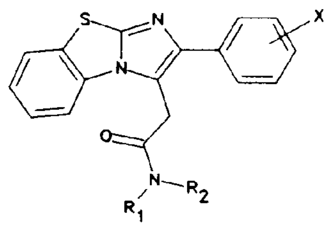

(I)

| N° | X | $R_1$ | $R_2$ | F (°C) |
|---|---|---|---|---|
| 1 | H | H | $CH_3$ | 260-263 |
| 2 | H | $CH_3$ | $CH_3$ | 206-208 |
| 3 | 4-F | H | $CH_3$ | 170-172 |
| 4 | 4-F | $CH_3$ | $CH_3$ | 230-231 |
| 5 | 2-Cl | H | $CH_3$ | 192-194 |
| 6 | 2-Cl | $CH_3$ | $CH_3$ | 159-162 |
| 7 | 3-Cl | H | $CH_3$ | 234-234,5 |
| 8 | 3-Cl | $CH_3$ | $CH_3$ | 156 |
| 9 | 4-Cl | H | $CH_3$ | 277-278 (d) |
| 10 | 4-Cl | $CH_3$ | $CH_3$ | 237-238 |
| 11 | 4-Br | H | $CH_3$ | 288-291 |
| 12 | 4-Br | $CH_3$ | $CH_3$ | 237-240 |
| 13 | 2-$CH_3$ | H | $CH_3$ | 162-165 |
| 14 | 2-$CH_3$ | $CH_3$ | $CH_3$ | 182-184 |
| 15 | 3-$CH_3$ | H | $CH_3$ | 201-203 |
| 16 | 3-$CH_3$ | $CH_3$ | $CH_3$ | 168-171 |
| 17 | 4-$CH_3$ | H | $CH_3$ | 259,5-261 |
| 18 | 4-$CH_3$ | $CH_3$ | $CH_3$ | 238-239 |
| 19 | 4-$OCH_3$ | H | $CH_3$ | 234-237 |
| 20 | 4-$OCH_3$ | $CH_3$ | $CH_3$ | 210-213 |
| 21 | H | H | H | 246-248 |

| N° | X | $R_1$ | $R_2$ | F (°C) |
|---|---|---|---|---|
| 22 | 4-Br | H | H | 287-290 |
| 23 | H | H | $CH_2CH_3$ | 232-235 |
| 24 | H | H | $CH_2CF_3$ | 293-295 |
| 25 | H | H | $(CH_2)_2CH_3$ | 200-202 |
| 26 | H | H | $CH(CH_3)_2$ | 274-275 |
| 27 | H | H | $CH_2CH(CH_3)_2$ | 207-208 |
| 28 | H | H | cyclo$C_5H_9$ | 242-244 |
| 29 | H | H | $CH_2-CH=CH_2$ | 210-212 |
| 30 | H | H | $CH_2-C\equiv CH$ | 246-248 |
| 31 | H | H | $(CH_2)_2OCH_3$ | 174-176,5 |
| 32 | H | H | $(CH_2)_2N(CH_3)_2$ | 132-135 |
| 33 | H | H | $C_6H_5$ | 224-226 |
| 34 | H | H | $CH_2C_6H_5$ | 236-238 |
| 35 | H | H | $(CH_2)_2C_6H_5$ | 190-192 |
| 36 | 4-Cl | H | $CH_2CH_3$ | 261-263 |
| 37 | 4-Cl | H | $(CH_2)_2CH_3$ | 249-251 |
| 38 | 2-$CH_3$ | H | $CH_2CH_3$ | 179-181 |
| 39 | 4-$CH_3$ | H | $CH_2CH_3$ | 246-248 |
| 40 | 4-$CH_3$ | H | $(CH_2)_2CH_3$ | 220-222 |
| 41 | 4-$CH_2CH_3$ | H | $CH_2CH_3$ | 225-227 |
| 42 | 4-$OCH_3$ | H | $CH_2CH_3$ | 224-225 |
| 43 | 4-$OCH_3$ | H | $(CH_2)_2CH_3$ | 210-212 |
| 44 | H | $CH_3$ | $(CH_2)_2CH_3$ | 110-111 |
| 45 | H | $CH_3$ | $C_6H_5$ | 156-157 |
| 46 | H | $CH_3$ | $CH_2C_6H_5$ | 155-156 |
| 47 | H | $CH_3$ | $(CH_2)_2C_6H_5$ | 147-148 |

| N° | X | $R_1$ | $R_2$ | F (°C) |
|---|---|---|---|---|
| 48 | H | $CH_2CH_3$ | $CH_2CH_3$ | 158-159 |
| 49 | H | $(CH_2)_2CH_3$ | $(CH_2)_2CH_3$ | 133-134 |
| 50 | H | $(CH_2)_3CH_3$ | $(CH_2)_3CH_3$ | 174-175 |
| 51 | H | 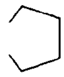 | | 205-206 |
| 52 | H | 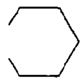 | | 151-152 |
| 53 | H | 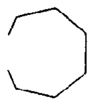 | | 174-175 |
| 54 | H | 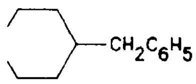 | | 154-155 |
| 55 | H | 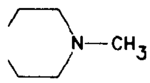 | | 195-196 |
| 56 | H | 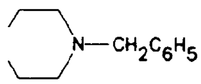 | | 193-194 |
| 57 | H | 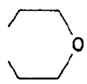 | | 207-208 |
| 58 | H | 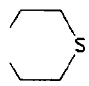 | | 177-178 |
| 59 | 4-Cl | 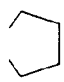 | | 248-249 |
| 60 | 4-Cl | 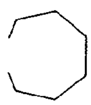 | | 183-184 |
| 61 | $2-CH_3$ | 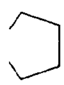 | | 246-247 |
| 62 | $2-CH_3$ | 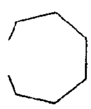 | | 165-166 |
| 63 | $4-CH_3$ | 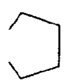 | | 215-216 |
| 64 | $4-CH_3$ | 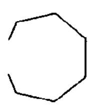 | | 156-157 |
| 65 | $4-OCH_3$ | 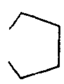 | | 236-237 |
| 66 | $4-OCH_3$ | 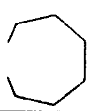 | | 198-199 |

| N° | X | $R_1$ | $R_2$ | F (°C) |
|---|---|---|---|---|
| 67 | 4-F | H | H | 268-270 (d) |
| 68 | 2-Cl | H | H | 203-206 |
| 69 | 3-Cl | H | H | 242-245 |
| 70 | 4-Cl | H | H | 288-290 (d) |
| 71 | 2-$CH_3$ | H | H | 203-206 |
| 72 | 3-$CH_3$ | H | H | 243-246 |
| 73 | 4-$CH_3$ | H | H | 253-254 |
| 74 | 4-$OCH_3$ | H | H | 285-289 (d) |
| 75 | 4-$C_2H_5$ | H | $CH_3$ | 218-220 |
| 76 | 4-$C_2H_5$ | $CH_3$ | $CH_3$ | 170-172 |
| 77 | 4-$C_2H_5$ | H | H | >250 |
| 78 | 4-$SCH_3$ | H | H | >250 |
| 79 | 4-$SCH_3$ | $CH_3$ | $CH_3$ | 160-162 |
| 80 | 4-$SCH_3$ | H | $CH_3$ | 250-252 |
| 81 | 4-$OC_2H_5$ | $CH_3$ | $CH_3$ | 174-176 |
| 82 | 4-$OC_2H_5$ | H | H | 265-266 |
| 83 | 4-$OC_2H_5$ | H | $CH_3$ | 238-240 |
| 84 | 4-$C_3H_7$ | $CH_3$ | $CH_3$ | 136-138 |
| 85 | 4-$C_3H_7$ | H | $CH_3$ | 214-216 |
| 86 | 4-$C_3H_7$ | H | H | 250-252 |
| 87 | 4-$SO_2CH_3$ | H | $CH_3$ | 232-234 |
| 88 | 4-$SO_2CH_3$ | H | H | 317-319 |
| 89 | 4-$SO_2CH_3$ | $CH_3$ | $CH_3$ | 202-204 |
| 90 | 4-CN | H | $CH_3$ | 278-286 (d) |
| 91 | 4-CN | $CH_3$ | $CH_3$ | 278-283 |
| 92 | 4-$CONH_2$ | H | $CH_3$ | 281-292 |
| 93 | 4-$CONH_2$ | H | H | >290 |
| 94 | 4-$CONH_2$ | $CH_3$ | $CH_3$ | 215-217 |

Le tableau 2 qui suit illustre les structures chimiques et les propriétés physiques de quelques composés de formule générale (IV), intermédiaires dans le procédé illustré par le schéma 1. Tous les composés sont à l'état de base. Dans la colonne "F (°C), "(d)" désigne un point de fusion avec décomposition.

Tableau 2

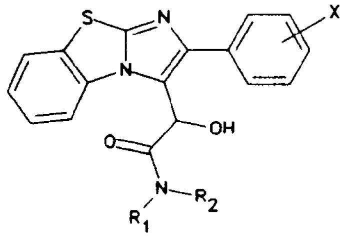

(IV)

| N° | X | $R_1$ | $R_2$ | F (°C) |
|---|---|---|---|---|
| 1' | H | H | H | 239-241 |
| 2' | H | H | $CH_3$ | 230-232 |
| 3' | H | $CH_3$ | $CH_3$ | 191-193 |
| 4' | 4-F | H | H | 267-269 |
| 5' | 4-F | H | $CH_3$ | 234-235 (d) |
| 6' | 4-F | $CH_3$ | $CH_3$ | 212-215 |
| 7' | 2-Cl | H | H | 244-246 |
| 8' | 2-Cl | H | $CH_3$ | 232-233 |
| 9' | 2-Cl | $CH_3$ | $CH_3$ | 203-204 |
| 10' | 3-Cl | H | H | 265-266 |
| 11' | 3-Cl | H | $CH_3$ | 228-229 |
| 12' | 3-Cl | $CH_3$ | $CH_3$ | 169-170 |
| 13' | 4-Cl | H | H | 263-266 |
| 14' | 4-Cl | H | $CH_3$ | 246-249 (d) |
| 15' | 4-Cl | $CH_3$ | $CH_3$ | 227-229 |
| 16' | 4-Br | H | H | 254-257 |

| N° | X | $R_1$ | $R_2$ | F (°C) |
|---|---|---|---|---|
| 17' | 4-Br | H | $CH_3$ | 261-264 |
| 18' | 4-Br | $CH_3$ | $CH_3$ | 222-225 |
| 19' | 2-$CH_3$ | H | H | 225-228 |
| 20' | 2-$CH_3$ | H | $CH_3$ | 221-224 |
| 21' | 2-$CH_3$ | $CH_3$ | $CH_3$ | 199-202 |
| 22' | 3-$CH_3$ | H | H | 253-255 |
| 23' | 3-$CH_3$ | H | $CH_3$ | 179-182 |
| 24' | 3-$CH_3$ | $CH_3$ | $CH_3$ | 177-181 |
| 25' | 4-$CH_3$ | H | H | 249-250 (d) |
| 26' | 4-$CH_3$ | H | $CH_3$ | 188-190 |
| 27' | 4-$CH_3$ | $CH_3$ | $CH_3$ | 205-206 |
| 28' | 4-$OCH_3$ | H | H | 234-237 |
| 29' | 4-$OCH_3$ | H | $CH_3$ | 229-231 |
| 30' | 4-$OCH_3$ | $CH_3$ | $CH_3$ | 225-227 |

Les composés de l'invention ont été soumis à des essais pharmacologiques qui ont mis en évidence leur intérêt comme substances à activités thérapeutiques.

Etude des liaisons membranaires vis-à-vis des récepteurs $\omega_1$ (benzodiazépiniques de type 1) et $\omega_2$(benzodiazépiniques de type 2).

L'affinité des composés pour les récepteurs $\omega_1$ du cervelet et $\omega_2$ de la moelle épinière a été déterminée selon une variante de la méthode décrite par S. Z. Langer et S. Arbilla dans *Fund. Clin. Pharmacol.*, **2,** 159-170 (1988), avec utilisation de $^3$H-flumazenil au lieu de $^3$H-diazepam comme radioligand. On homogénéise le tissu du cervelet ou de la moelle épinière pendant 60 s dans 120 ou 30 volumes, respectivement, de tampon glacé (50 mM Tris/HCl, pH 7,4, 120 mM NaCl, 5 mM KCl) puis, après dilution à 1/3, on fait incuber la suspension avec du $^3$H-flumazenil (activité spécifique 78 Ci/mmole, New England Nuclear) à une concentration de 1 nM et avec les composés de l'invention à différentes concentrations, dans un volume final de 525 µl. Après 30 minutes d'incubation à 0°C on filtre les échantillons sous vide sur des filtres Whatman GF/B® et on les lave immédiatement avec du tampon glacé. La liaison spécifique du $^3$H-flumazenil est déterminée en présence de diazépam 1 µM non marqué. On analyse les données selon les méthodes usuelles et on calcule la concentration $CI_{50}$, concentration qui inhibe de 50**%** la liaison du $^3$H-flumazenil. Les $CI_{50}$ des composés de l'invention se situent, dans ces essais, entre 0,1 et 1000 nM.

Etude de l'activité anticonvulsivante.

Activité vis-à-vis des convulsions maximales induites chez la souris par électrochoc ou par injection de pentétrazol.

Le protocole de cet essai est décrit par E. A. Swinyard et J. H. Woodhead dans *Antiepileptic Drugs*, Raven Press, New York, 111-126 (1982).
30 minutes après administration intrapéritonéale du composé à tester, on note le nombre de souris présentant des convulsions (extensions des pattes arrière), soit immédiatement après application d'un courant électrique (0,4 s, 60 mA, 50 Hz) à l'aide d'électrodes transcornéennes, soit pendant les 30 minutes qui suivent l'injection sous-cutanée de pentétrazol (125 mg/kg). Les résultats sont exprimés par la $DA_{50}$, dose qui protège 50% des animaux, calculée selon la méthode de J. T. Lichtfield et F. Wilcoxon (*J. Pharm. Exp. Ther.*, **96**, 99-113 (1949)) à partir de 3 ou 4 doses administrées chacune à un groupe de 8 à 10 souris.
Les $DA_{50}$ des composés de l'invention se situent, dans cet essai, entre 1 et 100 mg/kg par la voie intrapéritonéale.

Activité vis-à-vis des convulsions induites chez la souris par l'isoniazide.

L'activité intrinsèque des composés est déterminée par le temps de latence d'apparition des convulsions induites par l'administration sous-cutanée d'isoniazide (800 mg/kg) simultanément avec le composé à tester, injecté par voie intrapéritonéale, selon le protocole décrit par G. Perrault, E. Morel, D. Sanger et B. Zivkovic dans *Eur. J. Pharmacol.*, **156**, 189-196 (1988). Les résultats sont exprimés par la $DA_{50}$, dose qui produit 50% de l'effet maximal, par rapport aux animaux témoins, déterminée à partir de 3 ou 4 doses administrées chacune à un groupe de 8 à 10 souris.
Les $DA_{50}$ des composés de l'invention se situent, dans cet essai, entre 1 et 50 mg/kg par la voie intrapéritonéale et, selon les composés, l'effet maximal peut aller jusqu'à 350%.

Etude de l'activité anxiolytique.

L'activité anxiolytique est évaluée chez le rat dans le test de conflit de prise de boisson, selon la méthode décrite par J. R. Vogel, B. Beer et D. E. Clody dans *Psychopharmacologia* (Berl.), **21**, 1-7 (1971).
Après une diète hydrique de 48 heures, le rat est placé dans une enceinte isolée du bruit et équipée d'une pipette d'eau reliée à un anxiomètre délivrant un léger choc électrique tous les 20 coups de langue. Le nombre de chocs reçus est automatiquement compté pendant 3 minutes, et permet d'évaluer l'activité anxiolytique des composés testés. Les résultats sont exprimés par la dose efficace minimale (DEM), dose qui produit une augmentation significative du nombre de chocs reçus, par rapport au nombre observé chez les animaux témoins.
Les DEM des composés del'invention se situent, dans cet essai, entre 10 et 100 mg/kg par la voie intrapéritonéale ou orale.

Les résultats des essais effectués sur les composés de l'invention montrent que, *in vitro*, ils déplacent le $^3H$-flumazénil de ses sites de liaison spécifiques au niveau du cervelet et de la moelle épinière ; par conséquent ils présentent une affinité pour les sites $\omega_1$ et $\omega_2$ (benzodiazépiniques de type 1 et de type 2) situés au sein du complexe macromoléculaire $GABA_A$-sites $\omega$-canal chlorure.

*In vivo* ils se comportent comme des agonistes complets ou partiels, ou comme des antagonistes vis-à-vis de ces récepteurs.

Ils possèdent des propriétés anticonvulsivantes et anxiolytiques et, par conséquent, peuvent être utilisés pour le traitement d'affections liées aux désordres de la transmission GABAergique, tels que l'anxiété, les troubles du sommeil, l'épilepsie, la spasticité, les contractures musculaires, les troubles cognitifs, les troubles du sevrage vis-à-vis de l'alcoolisme, etc.

A cet effet ils peuvent être présentés sous toutes formes galéniques, associés à des excipients appropriés, pour l'administration entérale ou parentérale, par exemple sous forme de comprimés, dragées, gélules, capsules, solutions ou suspensions buvables ou injectables, suppositoires, etc, dosés pour permettre une administration journalière de 1 à 1000 mg de substance active.

## Revendications

1. Composé répondant à la formule générale (I)

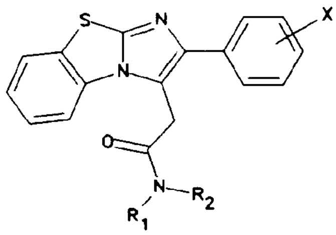

(I)

dans laquelle
X représente un atome d'hydrogène, de fluor, de chlore ou de brome, ou un groupe méthyle, éthyle, propyle, méthoxy, éthoxy, méthylthio, méthylsulfonyle, cyano ou aminocarbonyle,
$R_1$ représente un atome d'hydrogène ou un groupe alkyle en $C_1$-$C_4$, et
$R_2$ représente soit un atome d'hydrogène, soit un groupe alkyle en $C_1$-$C_5$ linéaire, ramifié ou cyclique, éventuellement substitué par un ou plusieurs atomes de fluor, par un groupe méthoxy, par un groupe diméthylamino ou par un groupe phényle,
soit un groupe prop-2-ényle, soit un groupe prop-2-ynyle,
soit un groupe phényle,
ou bien
$R_1$ et $R_2$ forment ensemble, et avec l'atome d'azote qui les porte, un groupe pyrrolidin-1-yle, pipéridin-1-yle, hexahydroazépin-1-yle, 4-(phénylméthyl)pipéridin-1-yle, 4-méthylpipérazin-1-yle, 4-(phénylméthyl)pipérazin-1-yle, morpholin-1-yle ou thiomorpholin-1-yle,
étant exclus les deux composés dans la formule desquels X réprésente un atome d'hydrogène ou de brome en position 4 et $R_1$ et $R_2$ représentent chacun un atome d'hydrogène,
à l'état de base libre ou de sel d'addition à un acide.

2. Procédé de préparation de composés selon la revendication 1, caractérisé en ce qu'on fait réagir un dérivé d'imidazo-[2,1-*b*]benzothiazole de formule générale (II)

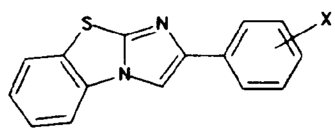

(II)

(dans laquelle X est tel que défini dans la revendication 1) avec l'acide glyoxylique dans un solvant protique, pour former un dérivé d'acide α-hydroxyacétique de formule générale (III)

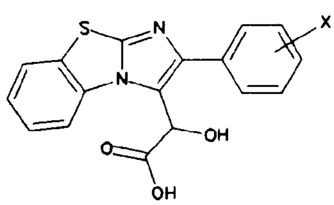

(III)

puis on fait réagir ce dernier avec l'anhydride acétique en présence d'une base organique, pour former le dérivé d'acide α-acétyloxyacétique correspondant, que l'on traite lui-même au moyen de *N,N'*-carbonyldiimidazole dans un solvant inerte, puis on traite *in situ* l'imidazolide intermédiaire ainsi obtenu anec une amine de formule générale $HNR_1R_2$ (dans laquelle $R_1$ et $R_2$ sont tels que définis ci-dessus) pour former un dérivé d'α-hydroxyacétamide de formule générale (IV)

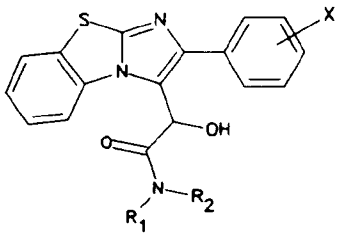

(IV)

qu'on traite ensuite avec un polyhalogénure d'acide sulfurique ou phosphorique, dans un solvant inerte, pour former un dérivé de α-halogénoacétamide de formule générale (V)

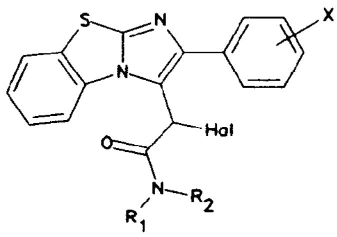

(V)

(dans laquelle X, $R_1$ et $R_2$ sont tels que définis ci-dessus et Hal représente un atome d'halogène, et finalement on fait réagir le composé de formule générale (V) soit avec un agent réducteur tel qu'un hydrure alcalin simple ou complexe, dans un solvant protique ou dans un solvant inerte miscible à l'eau, soit avec un agent réducteur tel qu'un hyposulfite ou un dithionite alcalin, ou encore avec l'hydroxyméthylsulfoxylate de sodium (Rongalite®), dans un solvant inerte chloré, éventuellement en présence d'un cosolvant inerte miscible à l'eau.

**3.** Composé répondant à la formule générale (IV)

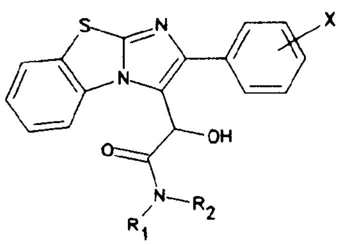

(IV)

dans laquelle X, $R_1$ et $R_2$ sont tels que définis dans la revendication 1,
à titre d'intermédiaire nécessaire dans le procédé selon la revendication 2.

**4.** Composé répondant à la formule générale (V)

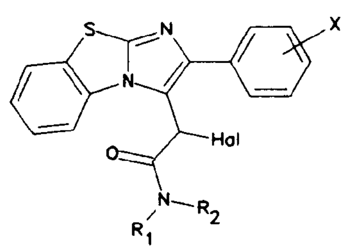

(V)

dans laquelle X, $R_1$ et $R_2$ sont tels que définis dans la revendication 1 et Hal représente un atome d'halogène,
à titre d'intermédiaire nécessaire dans le procédé selon la revendication 2.

**5.** Médicament caractérisé en ce qu'il consiste en un composé répondant à la formule générale (I)

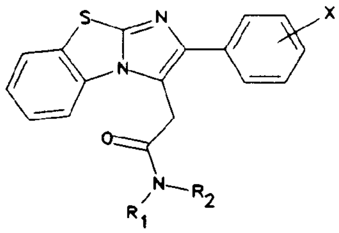

(I)

dans laquelle
X représente un atome d'hydrogène, de fluor, de chlore ou de brome, ou un groupe méthyle, éthyle, propyle, méthoxy, éthoxy, méthylthio, méthylsulfonyle, cyano ou aminocarbonyle,
$R_1$ représente un atome d'hydrogène ou un groupe alkyle en $C_1$-$C_4$, et
$R_2$ représente soit un atome d'hydrogène, soit un groupe alkyle en $C_1$-$C_5$ linéaire, ramifié ou cyclique, éventuellement substitué par un ou plusieurs atomes de fluor, par un groupe méthoxy, par un groupe diméthylamino ou par un groupe phényle,
soit un groupe prop-2-ényle, soit un groupe prop-2-ynyle,
soit un groupe phényle,
ou bien
$R_1$ et $R_2$ forment ensemble, et avec l'atome d'azote qui les porte, un groupe pyrrolidin-1-yle, pipéridin-1-yle, hexahydroazépin-1-yle, 4-(phénylméthyl)pipéridin-1-yle, 4-méthylpipérazin-1-yle, 4-(phénylméthyl)pipérazin-1-yle, morpholin-1-yle ou thiomorpholin-1-yle,
à l'état de base libre ou de sel d'addition à un acide.

**6.** Composition pharmaceutique caractérisée en ce qu'elle contient un composé tel que défini dans la revendication 5, associé à un excipient pharmaceutique.

**Revendication pour les Etats contractants suivants : ES, GR**

**1.** Procédé de préparation de composés répondant à la formule générale (I)

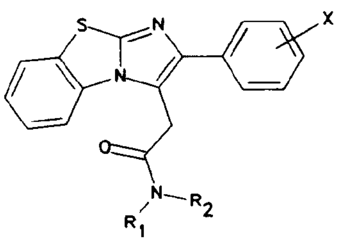

(I)

dans laquelle
X représente un atome d'hydrogène, de fluor, de chlore ou de brome, ou un groupe méthyle, éthyle, propyle, méthoxy, éthoxy, méthylthio, méthylsulfonyle, cyano ou aminocarbonyle,
$R_1$ représente un atome d'hydrogène ou un groupe alkyle en $C_1$-$C_4$, et
$R_2$ représente soit un atome d'hydrogène, soit un groupe alkyle en $C_1$-$C_5$ linéaire, ramifié ou cyclique, éventuellement substitué par un ou plusieurs atomes de fluor, par un groupe méthoxy, par un groupe diméthylamino ou par un groupe phényle,
soit un groupe prop-2-ényle, soit un groupe prop-2-ynyle,
soit un groupe phényle,
ou bien
$R_1$ et $R_2$ forment ensemble, et avec l'atome d'azote qui les porte, un groupe pyrrolidin-1-yle, pipéridin-1-yle, hexahydroazépin-1-yle, 4-(phénylméthyl)pipéridin-1-yle, 4-méthylpipérazin-1-yle, 4-(phénylméthyl)pipérazin-1-yle, morpholin-1-yle ou thiomorpholin-1-yle,
procédé caractérisé ce qu'on fait réagir un dérivé d'imidazo-[2,1-*b*]benzothiazole de formule générale (II)

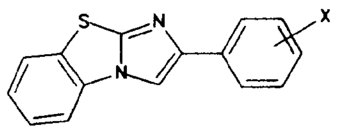

(II)

(dans laquelle X est tel que défini ci-dessus) avec l'acide glyoxylique dans un solvant protique, pour former un dérivé d'acide α-hydroxyacétique de formule générale (III)

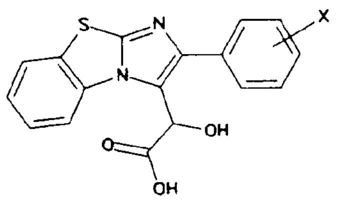

(III)

puis on fait réagir ce dernier avec l'anhydride acétique en présence d'une base organique, pour former le dérivé d'acide α-acétyloxyacétique correspondant, que l'on traite lui-même au moyen de *N,N'*-carbonyldiimidazole dans un solvant inerte, puis on traite *in situ* l'imidazolide intermédiaire ainsi obtenu anec une amine de formule générale $HNR_1R_2$ (dans laquelle $R_1$ et $R_2$ sont tels que définis ci-dessus) pour former un dérivé d'α-hydroxyacétamide de formule générale (IV)

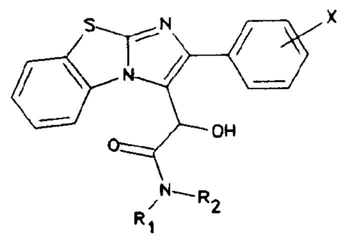

(IV)

qu'on traite ensuite avec un polyhalogénure d'acide sulfurique ou phosphorique, dans un solvant inerte, pour former un dérivé de α-halogénoacétamide de formule générale (V)

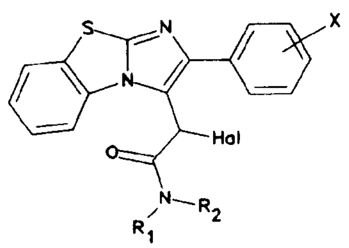

(V)

(dans laquelle X, $R_1$ et $R_2$ sont tels que définis ci-dessus et Hal représente un atome d'halogène, et finalement on fait réagir le composé de formule générale (V) soit avec un agent réducteur tel qu'un hydrure alcalin simple ou complexe, dans un solvant protique ou dans un solvant inerte miscible à l'eau, soit avec un agent réducteur tel qu'un hyposulfite ou dithionite alcalin, ou encore avec l'hydroxyméthylsulfoxylate de sodium (Rongalite®), dans un solvant inerte chloré, éventuellement en présence d'un cosolvant inerte miscible à l'eau.

Office européen des brevets

# RAPPORT DE RECHERCHE EUROPEENNE

Numero de la demande
EP 92 40 1956

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,X | CHEMICAL AND PHARMACEUTICAL BULLETIN. vol. 36, no. 12, Décembre 1988, TOKYO JP pages 4760 - 4768; A. N. EL-SHORBAGI ET AL.: 'Imidazo(2,1-b)benzothiazoles. I' * Abrégé; page 4762, composés 6a-b * | 1,5,6 | C07D513/04 A61K31/425 //(C07D513/04, 277:00,235:00) |
| A | CHEMICAL AND PHARMACEUTICAL BULLETIN. vol. 37, no. 11, Novembre 1989, TOKYO JP pages 2971 - 2975; A. B. EL-SHORBAGI ET AL.: 'Imidazo(2,1-b)benzothiazoles. II. Synthesis and antiinflammatory activity of some imidazo(2,1-b)benzothiazoles' * Abrégé; page 2973, composés 7a-e * | 1,3,5,6 | |
| A | EP-A-0 313 458 (ROUSSEL - UCLAF) * page 7, ligne 49 - page 8, ligne 37; revendications 1,12 * | 1,5,6 | |
| A | FR-A-2 646 158 (ROUSSEL-UCLAF) * page 4, ligne 27 - page 4, ligne 30; revendications 1,3,8,10 * | 1,5,6 | **DOMAINES TECHNIQUES RECHERCHES (Int. Cl.5)** |
| A | EP-A-0 082 712 (YAMANOUCHI PHARMACEUTICAL) * revendications 1,10 * | 1,5,6 | C07D A61K |
| A | EP-A-0 347 880 (NIKKEN CHEMICALS) * revendications 1,13,14 * | 1,5,6 | |
| D,A | JOURNAL OF MEDICINAL CHEMISTRY. vol. 29, no. 3, 1986, WASHINGTON US pages 386 - 394; T. MASE ET AL.: 'Imidazo(2,1-b)benzothiazoles. 2. New immunosuppressive agents.' * page 386, abrégé; tableau I * | 1,5,6 | |

Le présent rapport a été établi pour toutes les revendications

| Lieu de la recherche | Date d'achèvement de la recherche | Examinateur |
|---|---|---|
| LA HAYE | 21 SEPTEMBRE 1992 | VOYIAZOGLOU D. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)